# EUROPEAN PATENT APPLICATION

(11) **EP 4 299 722 A1**
(43) Date of publication of application: **03.01.2024**
(21) Application number: 23181205.8
(22) Date of filing: 23.06.2023
(51) Int. Cl.: C12N 5/0783

(54) **METHOD FOR INDUCING ANTIGEN-SPECIFIC T CELL AND USE THEREOF IN TREATING CANCER OR VIRUS INFECTION**

(30) Priority: 23.06.2022 US 202263354868 P
(71) Applicant: Fullhope Biomedical Co., Ltd., New Taipei City 24159 (TW)
(72) Inventor: LEE, Jan-Mou, 24159 New Taipei City (TW); FANG, Chih-Hao, 24159 New Taipei City (TW); CHENG, Ya-Fang, 24159 New Taipei City (TW)
(74) Representative: Engelhard, Markus

(57) **Abstract**

The disclosure relates to a method for inducing antigen-specific T cell and the use thereof in treating cancer or virus infection. The method comprises culturing PBMCs with antigenic peptide, IL-2, IL-15 and IFN-α.

## Description

### FIELD OF INVENTION

The disclosure relates to a method for inducing antigen-specific T cell and the use thereof in treating cancer or virus infection.

### BACKGROUND OF THE INVENTION:

Cancer is usually treated with surgery, radiation therapy, or chemotherapy. Surgery for cancer will cause physical damage to the body, such as wounds, making it difficult to recuperate after surgery. Although radiation therapy does not cause wounds, radiation may remain in the body or damage non-cancerous cells, causing lasting damage. Chemotherapy can also cause harm to the body by killing cancer cells and affecting other normal cells.

Immune surveillance plays a critical role against cancer and represents a very attractive therapeutic approach, especially in light of the many shortcomings of conventional surgery, radiation and chemotherapies in the management of cancer.

In cancer immunotherapy, the immune system is used passively or actively to specifically target and kill cancer cells. Immunotherapy offers target specificity that eliminates off-target toxicity while still eliciting potent anticancer responses. In passive immunotherapy, by targeting tumor cells or their microenvironment, endogenous anti-tumor immune responses can be enhanced and tumor cell growth inhibited. In active immunotherapy, immune cells are stimulated to fight with cancer.

Active immunotherapy is highly dependent on efficient stimulation of antigen-specific immune cells, such as killer T cells and antibody-producing B cells. How to induce T cells to produce antigen specificity is an important topic of concern at present. The present disclosure provides a method for inducing antigen-specific T cell and the use thereof in treating cancer or virus infection.

### SUMMARY OF THE INVENTION

In view of the urgent need of the art, provided herein are method of inducing an antigen-specific T cell that are safe and effective in the treatment of the cancer. In addition, the antigen-specific T cells are easy to manufacture with low cost and high yield.

In one embodiment, the disclosure provides a method for of inducing an antigen-specific T cell, comprising:
culturing a peripheral blood mononuclear cell (PBMC) in a first culture medium comprising an antigenic peptide, IL-2, IL-15 and IFN-α to obtain a first cell population;
culturing the first cell population in a second culture medium comprising IL-2, IL-15 and IFN-α to obtain a second cell population; and
culturing the second cell population in a third culture medium comprising IL-2 and IL-15 to obtain the antigen-specific T cell.

In one embodiment, the antigenic peptide is from a cancer or a virus in a concentration of 1 µg/mL to 1 mg/mL, preferably 5 µg/mL to 500 µg/mL, preferably 10 µg/mL to 100 µg/mL or more preferably 10 µg/mL.

In one embodiment, the cancer comprises nasopharyngeal cancer, bladder cancer, biliary cancer, bone cancer, brain tumor, breast cancer, cervical cancer, colorectal cancer, colon cancer, esophageal cancer, epidermal carcinoma, gastric cancer, gastrointestinal stromal tumor (GIST), glioma, hematopoietic tumors of lymphoid lineage, hepatic cancer, non-Hodgkin's lymphoma, Kaposi's sarcoma, leukemia, lung cancer, lymphoma, intestinal cancer, melanoma, myeloid leukemia, pancreatic cancer, prostate cancer, retinoblastoma, ovary cancer, renal cell carcinoma, spleen cancer, squamous cell carcinoma, thyroid cancer, or thyroid follicular cancer.

In one embodiment, the cancer is Epstein-Barr virus (EBV)-associated cancer.

In one embodiment, the cancer is EBV-positive gastric cancer, EBV-positive cervical cancer, EBV-positive Burkitt's lymphoma, EBV-positive T cell lymphoma, EBV-positive breast cancer, EBV-positive leiomyosarcoma, EBV-positive smooth muscle tumor, EBV-positive Hodgkin lymphoma, EBV-positive nasopharyngeal cancer, or EBV-positive post-transplant lymphoproliferative disorder (PTLD).

In one embodiment, the antigenic peptide comprising BMI,F-1, EBV peptide pool or CMV peptide pool.

In one embodiment, the first culture medium, the second culture medium and the third culture medium is cultured for 1-3 days, respectively.

In one embodiment, the IL-2 is in a concentration from 1 to 500 ng/mL, preferably 5 to 200 ng/mL, 10 to 100 ng/mL, 20 to 80 ng/mL, more preferably 40 ng/mL.

In one embodiment, the IL-15 is in a concentration from 1 to 100 ng/mL, preferably 2 to 50 ng/mL, 2.5 to 10 ng/mL, more preferably 2.5 ng/mL.

In one embodiment, the IFN-α is in a concentration from 1 to 100 ng/mL, preferably 2 to 50 ng/mL, 3 to 25 ng/mL, 4 to 10 ng/mL, more preferably 5 ng/mL.

In one embodiment, the first culture medium, the second culture medium and the third culture medium further comprises AIM-V medium, respectively.

In one embodiment, the first culture medium, the second culture medium and the third culture medium further comprises platelet lysate, preferably human platelet lysate (HPL), in a concentration of 1 to 10 wt% (based on the culture medium), preferably 2 to 8 wt% (based on the culture medium), 3 to 6 wt% (based on the culture medium), more preferably 4 wt% (based on the culture medium).

Some embodiment of the present disclosure provides a use of a composition comprising a therapeutically effective amount of the antigen-specific T cell and a pharmaceutically acceptable carrier for preventing or treating cancer or virus infection in a subject in need thereof.

Still embodiment of the present disclosure provides a use of a composition comprising a therapeutically effective amount of the antigen-specific T cell and a pharmaceutically acceptable carrier for manufacture of a medicament for preventing or treating cancer or virus infection in a subject in need thereof.

The present disclosure further provides a composition for use in preventing or treating cancer or virus infection in a subject in need thereof, comprising a therapeutically effective amount of the antigen-specific T cell and a pharmaceutically acceptable carrier.

### BRIEF DESCRIPTION OF THE DRAWINGS

Fig. 1 illustrates T cell growth cytokines promoted BMI,F1-induced EBV specific CD8 T cell activation and expansion.
Figs. 2 to 4 illustrate addition of IFN-α on day 0 and day 3 promoted 4-1BB⁺CD8 T cells expansion.
Figs. 5 and 6 illustrate Addition of IFN-α on day 0 and day 3 promoted 4-1BB⁺BMLF1-specific CD8 T cells expansion.
Fig. 7 illustrates nine-day culture period revealed the best 4-1BB⁺BMLF1-specific CD8 T cell expansion.
Fig. 8 illustrates addition of IFN-α on day 0 and day 3 displayed the best T-dependent cytotoxicity of 4-1BB⁺BMLF1-specific CD8 T cells.
Fig. 9 illustrates different IL-2 synergized with IL-15 to promote BMLF1-induced EBV specific CD8 T cell responses.
Fig. 10 illustrates phenotypic analysis IFN-α induced EBV specific T cells (EBaT8).
Fig. 11 illustrates functionality analysis of EBaT8. Evaluation of cytotoxicity of EBaT8 was performed by PanToxilux kit (OncoImmunin, Inc.).
Figs. 12 and 13 illustrate that PepTivator EBV peptide pool is capable of inducing EBV-specific T cell (EBaT8) expansion in the presence of IFN-α.
Fig. 14 illustrates that EBV-specific T cells (EBaT8) generated from PepTivator EBV peptide pool and IFN-α exhibited T-dependent cytotoxicity.
Figs. 15 and 16 illustrate that PepTivator CMV peptide pool is capable of inducing CMV-specific T cell expansion in the presence of IFN-α (cell harvested on day 6).
Fig. 17 illustrates that CMV-specific T cells generated from PepTivator CMV peptide pool and IFN-α exhibited T-dependent cytotoxicity (cell harvested on day 6).

### DETAILED DESCRIPTION

The foregoing and other aspects of the present disclosure will now be described in more detail with respect to other embodiments described herein. It should be appreciated that the invention can be embodied in different forms and should not be construed as limited to the embodiments set forth herein. Rather, these embodiments are provided so that this disclosure will be thorough and complete, and will fully convey the scope of the invention to those skilled in the art.

The terminology used in the description of the invention herein is for the purpose of describing particular embodiments only and is not intended to be limiting of the invention. As used in the description of the invention and the appended claims, the singular forms "a", "an" and "the" are intended to include the plural forms as well, unless the context clearly indicates otherwise.

As used herein, the terms "comprises," "comprising," "includes," "including," "has," "having," "contains", "containing," "characterized by" or any other variation thereof, are intended to cover a non-exclusive inclusion, subject to any limitation explicitly indicated. For example, a composition, mixture, process or method that comprises a list of elements is not necessarily limited to only those elements but may include other elements not expressly listed or inherent to such composition, mixture, process, or method.

The transitional phrase "consisting of" excludes any elements, steps, or ingredients not specified. If in the claim, such would close the claim to the inclusion of materials other than those recited except for impurities ordinarily associated therewith. When the phrase "consisting of" appears in a clause of the body of a claim, rather than immediately following the preamble, it limits only the element set forth in that clause; other elements are not excluded from the claim as a whole.

Where applicants have defined an invention or a portion thereof with an openended term such as "comprising," it should be readily understood that (unless otherwise stated) the description should be interpreted to also describe such an invention using the term "consisting of."

All numbers herein may be understood as modified by "about." As used herein, the term "about" is used to indicate that a value includes for example, the inherent variation of error for a measuring device, the method being employed to determine the value, or the variation that exists among the study subjects. Typically the term is meant to encompass approximately or less than 1%, 2%, 3%, 4%, 5%, 6%, 7%, 8%, 9%, 10%, 11%, 12%, 13%, 14%, 15%, 16%, 17%, 18%, 19% or 20% variability depending on the situation.

The use of the term "or" in the claims is used to mean "and/or" unless explicitly indicated to refer to alternatives only or the alternatives are mutually exclusive, although the disclosure supports a definition that refers to only alternatives and "and/or."

"Subject" as used herein refers to animals, including, for example, a mammalian subject diagnosed with or suspected of having or developing cancer(s). In an embodiment, the term "subject" can refer to a vertebrate having cancer or to a vertebrate deemed to be in need of cancer treatment. Subjects include warm-blooded animals, such as mammals, such as a primate, and, more preferably, a human. Non-human primates are subjects as well. The term subject includes domesticated animals, such as cats, dogs, apes, etc., livestock (for example, cattle, horses, pigs, sheep, goats, etc.) and laboratory animals (for example, mouse, rabbit, rat, gerbil, guinea pig, etc.). Thus, veterinary uses and medical formulations are contemplated herein.

"Administering" or "Administration" is referred to herein as providing an antigen-specific T cell or a pharmaceutical composition of the present application to a subject. By way of example and not limitation, administration may be performed via parenteral, subcutaneous, intramuscular, intravenous, intra-articular, intrabronchial, intraabdominal, intracapsular, intracartilaginous, intracavitary, intracelial, intracerebellar, intracerebroventricular, intracolic, intracervical, intragastric, intrahepatic, intramyocardial, intraosteal, intrapelvic, intrapericardiac, intraperitoneal, intrapleural, intraprostatic, intrapulmonary, intrarectal, intrarenal, intraretinal, intraspinal, intrasynovial, intrathoracic, intrauterine, intravesical, bolus, vaginal, rectal, buccal, sublingual, intranasal, and transdermal. For example, injection may be performed by intravenous (*i.v*.) injection, subcutaneous (*s.c.*) injection, intradermal (*i*.*d*) injection, intraperitoneal (*i.p*.) injection, or intramuscular (*i*.*m*.) injection. One or more such routes may be employed. Parenteral administration can be, for example, by bolus injection or by gradual perfusion over time. Alternatively, or concurrently, administration may be by the oral route.

The use of the terms "treating," or "treatment" is referred to herein as administration of an antigen-specific T cell or a pharmaceutical composition to a subject with the purpose to cure, alleviate, relieve, remedy, prevent, or ameliorate a disorder, symptoms of the disorder, a disease state secondary to the disorder, or predisposition toward the disorder. The terms "inhibiting," "reducing," or "prevention," or any variation of these terms, when used in the claims and/or the specification includes any measurable decrease or complete inhibition to achieve a desired result.

"Cancer(s)" that may be treated by the pharmaceutical composition of the application include those classified by site include cancer of the oral cavity and pharynx (lip, tongue, salivary gland, floor of mouth, gum and other mouth, nasopharynx, tonsil, oropharynx, hypopharynx, other oral/pharynx); cancers of the digestive system (esophagus; stomach; small intestine; colon and rectum; anus, anal canal, and anorectum; liver; intrahepatic bile duct; gallbladder; other biliary; pancreas; retroperitoneum; peritoneum, omentum, and mesentery; other digestive); cancers of the respiratory system (nasal cavity, middle ear, and sinuses; larynx; lung and bronchus; pleura; trachea, mediastinum, and other respiratory); cancers of the mesothelioma; bones and joints; and soft tissue, including heart; skin cancers, including melanomas and other non-epithelial skin cancers; Kaposi's sarcoma and breast cancer; cancer of the female genital system (cervix uteri; corpus uteri; uterus, ovary; vagina; vulva; and other female genital); cancers of the male genital system (prostate gland; testis; penis; and other male genital); cancers of the urinary system (urinary bladder; kidney and renal pelvis; ureter; and other urinary); cancers of the eye and orbit; cancers of the brain and nervous system (brain; and other nervous system); cancers of the endocrine system (thyroid gland and other endocrine, including thymus); lymphomas (Hodgkin's disease and non-Hodgkin's lymphoma), multiple myeloma, and leukemias (lymphocytic leukemia; myeloid leukemia; monocytic leukemia; and other leukemias).

Other cancers, classified by histological type, that may be suitable targets for the therapeutic compositions according to the present application include, but are not limited to, neoplasm, malignant; Carcinoma, not otherwise specified (NOS); Carcinoma, undifferentiated, NOS; Giant and spindle cell carcinoma; Small cell carcinoma, NOS; Papillary carcinoma, NOS; Squamous cell carcinoma, NOS; Lymphoepithelial carcinoma; Basal cell carcinoma, NOS; Pilomatrix carcinoma; Transitional cell carcinoma, NOS; Papillary transitional cell carcinoma; Adenocarcinoma, NOS; Gastrinoma, malignant; Cholangiocarcinoma; Hepatocellular carcinoma, NOS; Combined hepatocellular carcinoma and cholangiocarcinoma; Trabecular adenocarcinoma; Adenoid cystic carcinoma; Adenocarcinoma in adenomatous polyp; Adenocarcinoma, familial polyposis coli; Solid carcinoma, NOS; Carcinoid tumor, malignant; Bronchioloalveolar adenocarcinoma; Papillary adenocarcinoma, NOS; Chromophobe carcinoma; Acidophil carcinoma; Oxyphilic adenocarcinoma; Basophil carcinoma; Clear cell adenocarcinoma, NOS; Granular cell carcinoma; Follicular adenocarcinoma, NOS; Papillary and follicular adenocarcinoma; Nonencapsulating sclerosing carcinoma; Adrenal cortical carcinoma; Endometroid carcinoma; Skin appendage carcinoma; Apocrine adenocarcinoma; Sebaceous adenocarcinoma; Ceruminous adenocarcinoma; Mucoepidermoid carcinoma; Cystadenocarcinoma, NOS; Papillary cystadenocarcinoma, NOS; Papillary serous cystadenocarcinoma; Mucinous cystadenocarcinoma, NOS; Mucinous adenocarcinoma; Signet ring cell carcinoma; Infiltrating duct carcinoma; Medullary carcinoma, NOS; Lobular carcinoma; Inflammatory carcinoma; Paget's disease, mammary; Acinar cell carcinoma; Adenosquamous carcinoma; Adenocarcinoma w/ squamous metaplasia; Thymoma, malignant; Ovarian stromal tumor, malignant; Thecoma, malignant; Granulosa cell tumor, malignant; Androblastoma, malignant; Sertoli cell carcinoma; Leydig cell tumor, malignant; Lipid cell tumor, malignant; Paraganglioma, malignant; Extra-mammary paraganglioma, malignant; Pheochromocytoma; Glomangiosarcoma; Malignant melanoma, NOS; Amelanotic melanoma; Superficial spreading melanoma; Malig melanoma in giant pigmented nevus; Epithelioid cell melanoma; Blue nevus, malignant; Sarcoma, NOS; Fibrosarcoma, NOS; Fibrous histiocytoma, malignant; Myxosarcoma; Liposarcoma, NOS; Leiomyosarcoma, NOS; Rhabdomyosarcoma, NOS; Embryonal rhabdomyosarcoma; Alveolar rhabdomyosarcoma; Stromal sarcoma, NOS; Mixed tumor, malignant, NOS; Mullerian mixed tumor; Nephroblastoma; Hepatoblastoma; Carcinosarcoma, NOS; Mesenchymoma, malignant; Brenner tumor, malignant; Phyllodes tumor, malignant; Synovial sarcoma, NOS; Mesothelioma, malignant; Dysgerminoma; Embryonal carcinoma, NOS; Teratoma, malignant, NOS; Struma ovarii, malignant; Choriocarcinoma; Mesonephroma, malignant; Hemangiosarcoma; Hemangioendothelioma, malignant; Kaposi's sarcoma; Hemangiopericytoma, malignant; Lymphangiosarcoma; Osteosarcoma, NOS; Juxtacortical osteosarcoma; Chondrosarcoma, NOS; Chondroblastoma, malignant; Mesenchymal chondrosarcoma; Giant cell tumor of bone; Ewing's sarcoma; Odontogenic tumor, malignant; Ameloblastic odontosarcoma; Ameloblastoma, malignant; Ameloblastic fibrosarcoma; Pinealoma, malignant; Chordoma; Glioma, malignant; Ependymoma, NOS; Astrocytoma, NOS; Protoplasmic astrocytoma; Fibrillary astrocytoma; Astroblastoma; Glioblastoma, NOS; Oligodendroglioma, NOS; Oligodendroblastoma; Primitive neuroectodermal; Cerebellar sarcoma, NOS; Ganglioneuroblastoma; Neuroblastoma, NOS; Retinoblastoma, NOS; Olfactory neurogenic tumor; Meningioma, malignant; Neurofibrosarcoma; Neurilemmoma, malignant; Granular cell tumor, malignant; Malignant lymphoma, NOS; Hodgkin's disease, NOS; Hodgkin's; paragranuloma, NOS; Malignant lymphoma, small lymphocytic; Malignant lymphoma, large cell, diffuse; Malignant lymphoma, follicular, NOS; Mycosis fungoides; Other specified non-Hodgkin's lymphomas; Malignant histiocytosis; Multiple myeloma; Mast cell sarcoma; Immunoproliferative small intestinal disease; Leukemia, NOS; Lymphoid leukemia, NOS; Plasma cell leukemia; Erythroleukemia; Lymphosarcoma cell leukemia; Myeloid leukemia, NOS; Basophilic leukemia; Eosinophilic leukemia; Monocytic leukemia, NOS; Mast cell leukemia; Megakaryoblastic leukemia; Myeloid sarcoma; and Hairy cell leukemia.

An "effective amount," as used herein, refers to a dose of the modified antigen-specific T cell or pharmaceutical composition that is sufficient to reduce the symptoms and signs of cancer, which include, but are not limited to, weight loss, pain or tumor mass which is detectable, either clinically as a palpable mass or radiologically through various imaging means.

In certain embodiments, it is desired to limit, reduce, or ameliorate the size of tumor or cancer lesions. The routes of administration will vary, naturally, with the location and nature of the lesion or site to be targeted, and include, e.g., regional, parenteral, intravenous, intramuscular, and/or systemic administration and formulation. Direct injection or injection into the vasculature or the vessels to and from and within an organ or tissue is specifically contemplated for target areas. Local, regional, or systemic administration also may be appropriate.

In the disclosure herein, the expression level or surface density of the cell surface antigen using FACS/flow cytometry analysis are defined in Table 1. The interpretation for the various expression levels in Table 1 is an example of defining the expression level of the cell surface antigen. It should be noted that the flow cytometry signal level intensity varies with the following factors: the flow cytometry, the software and different batches of antibody used.

**Table 1**

| Symbol | Interpretation |
|---|---|
| - | Flow cytometry signal level intensity less than or equal to 10° (*i.e.,* 1) |
| + (Dim) | Flow cytometry signal level intensity between 10⁰ to 10¹ |
| + | Flow cytometry signal level intensity between 10¹ to 10² |
| + (hi) | Flow cytometry signal level intensity greater than or equal to 10² |

Unless otherwise defined, all technical and scientific terms used herein have the same meaning as commonly understood by one of ordinary skill in the art to which this invention belongs. All publications, patent applications, patents and other references cited herein are incorporated by reference in their entireties for the teachings relevant to the sentence and/or paragraph in which the reference is presented.

Type I interferons are well known that can regulate T cell function. For example:
1. In-sequence signaling: Concomitant or slightly delayed type I IFN signaling relative to TCR engagement promotes survival, effector cell differentiation.
2. Out-of-sequence signaling: Pre-exposure of T cells to type I IFNs in relation to TCR engagement induces a STAT1-dependent anti-proliferative and pro-apoptotic program.

The present disclosure provides a method for of inducing an antigen-specific T cell, comprising: culturing a peripheral blood mononuclear cell (PBMC) in a first culture medium comprising an antigenic peptide, IL-2, IL-15 and IFN-α to obtain a first cell population; culturing the first cell population in a second culture medium comprising IL-2, IL-15 and IFN-α to obtain a second cell population; and culturing the second cell population in a third culture medium comprising IL-2 and IL-15 to obtain the antigen-specific T cell.

The antigenic peptide may be in a concentration of 1 µg/mL to 1 mg/mL, preferably 5 µg/mL to 500 µg/mL, preferably 10 µg/mL to 100 µg/mL or more preferably 10 µg/mL.

The antigenic peptide may be from a cancer or a virus. The cancer may comprises nasopharyngeal cancer, bladder cancer, biliary cancer, bone cancer, brain tumor, breast cancer, cervical cancer, colorectal cancer, colon cancer, esophageal cancer, epidermal carcinoma, gastric cancer, gastrointestinal stromal tumor (GIST), glioma, hematopoietic tumors of lymphoid lineage, hepatic cancer, non-Hodgkin's lymphoma, Kaposi's sarcoma, leukemia, lung cancer, lymphoma, intestinal cancer, melanoma, myeloid leukemia, pancreatic cancer, prostate cancer, retinoblastoma, ovary cancer, renal cell carcinoma, spleen cancer, squamous cell carcinoma, thyroid cancer, or thyroid follicular cancer.

The cancer may be Epstein-Barr virus (EBV)-associated cancer. The cancer may be EBV-positive gastric cancer, EBV-positive cervical cancer, EBV-positive Burkitt's lymphoma, EBV-positive T cell lymphoma, EBV-positive breast cancer, EBV-positive leiomyosarcoma, EBV-positive smooth muscle tumor, EBV-positive Hodgkin lymphoma, EBV-positive nasopharyngeal cancer, or EBV-positive post-transplant lymphoproliferative disorder (PTLD).

The antigenic peptide may comprise BMI,F-1 or EBV peptide pool. The peptide also may be from Cytomegalovirus (CMV) peptide pool or other tumor-associated antigens.

The first culture medium, the second culture medium and the third culture medium may be cultured for 1-3 days, respectively. On the other hand, the IL-2 may be in a concentration from 1 to 500 ng/mL, preferably 5 to 200 ng/mL, 10 to 100 ng/mL, 20 to 80 ng/mL, more preferably 40 ng/mL. Further, the IL-15 may be in a concentration from 1 to 100 ng/mL, preferably 2 to 50 ng/mL, 2.5 to 10 ng/mL, more preferably 2.5 ng/mL. Still further, the IFN-α is in a concentration from 1 to 100 ng/mL, preferably 2 to 50 ng/mL, 3 to 25 ng/mL, 4 to 10 ng/mL, more preferably 5 ng/mL.

The first culture medium, the second culture medium and the third culture medium may further comprise AIM-V medium. The first culture medium, the second culture medium and the third culture medium may further comprise platelet lysate, preferably human platelet lysate (HPL), in a concentration of 1 to 10 wt% (based on the culture medium), preferably 2 to 8 wt% (based on the culture medium), 3 to 6 wt% (based on the culture medium), more preferably 4 wt% (based on the culture medium).

A composition comprising a therapeutically effective amount of the antigen-specific T cell may be manufactured by above-mentioned method, and the composition may be combined with a pharmaceutically acceptable carrier. The composition may be used for preventing or treating cancer or virus infection in a subject in need thereof. Further, the composition may be used for manufacture of a medicament for preventing or treating cancer or virus infection in a subject in need thereof.

The following examples will show the different strategy of inducing antigen-T cells and finally find a preferable procedure thereof.

### Example 1

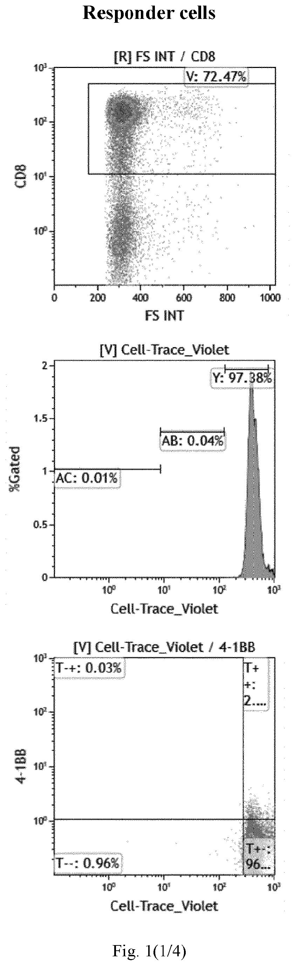

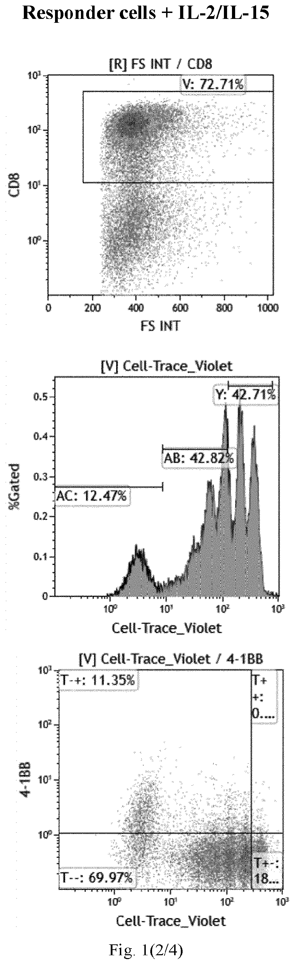

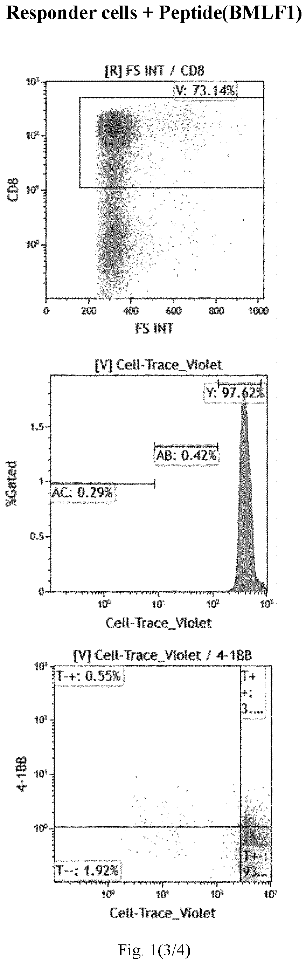

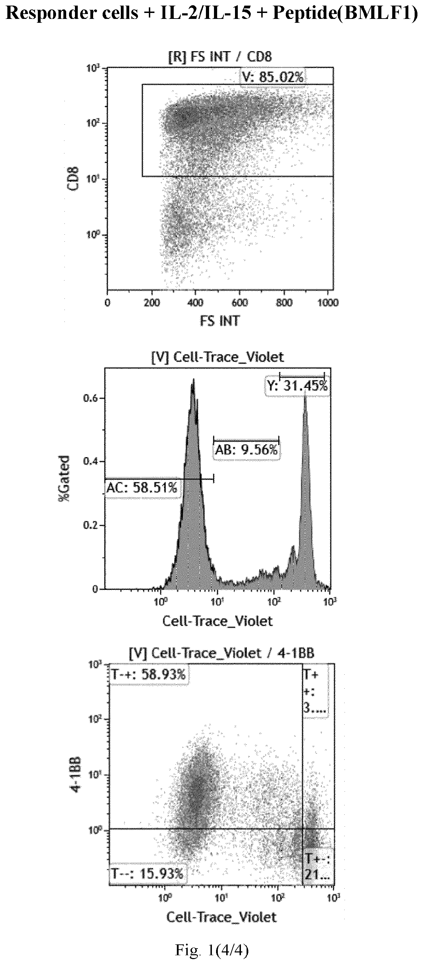

Please see Fig. 1. Fig. 1 illustrates T cell growth cytokines promoted BMLF1-induced EBV specific CD8 T cell activation and expansion. Responder PBMCs were labeled with Cell-Trace Violet, then cultured in AIM-V medium and 4% HPL for 9 days. In each particular group, responder PBMCs were respectively cultured in the presence of hIL-2 (40 ng/ml) and hIL-15 (2.5 ng/ml), BMI,F1 peptide (10 µg/ml), and BMLF1 peptide (10 µg/ml) plus hIL-2 (40 ng/ml) and hIL-15 (2.5 ng/ml). On day 9, total cells were harvested and stained with mAbs against 4-1BB-PE, CD8-APC-Alexa Flour 700, CD56-APC-Cy7, CD14-APC-Alexa Flour 750, CD19APC-Alexa Flour 750, and CD3-Krome Orange. Samples acquisition via Navios Flow Cytometer and data analysis via Kaluza software.

As shown in Fig. 1, the IL-2 and IL-15 may induce differentiation of PBMCs. The example of peptide (BMLF1) does not induce the differentiation of PBMCs. The combination of IL-2 and IL-15 and BMLF1 shows significant differentiation of PBMCs. The example induced by BMI,F-1 only cannot effectively promote the replication of CD8 T cells because the TCR threshold is too high. However, the example induced by IL-2/IL-15 has the function of reducing the TCR threshold, allowing T cells to replicate. Therefore, with the help of IL-2/IL-15, it contributes to the replication of BMLF peptide-induced CD8 T cells and the expression of 4-1BB. As the result, the IL-2 and IL-15 may have a function to induce T cell differentiation individually, but will perform better with the combination with the antigen-peptide of BMLF1.

### Example 2

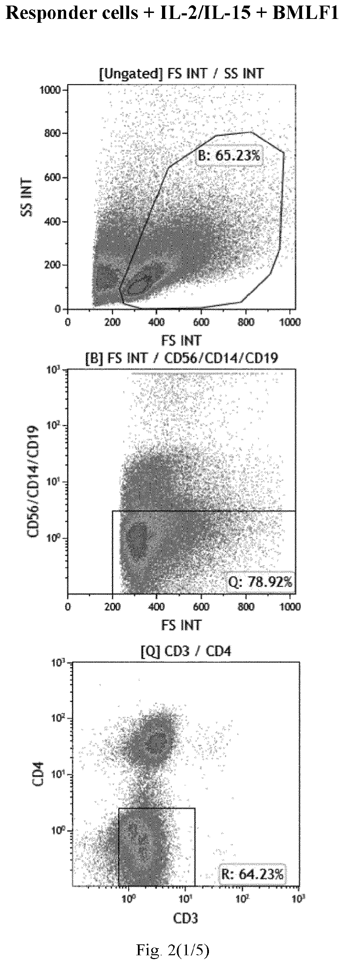

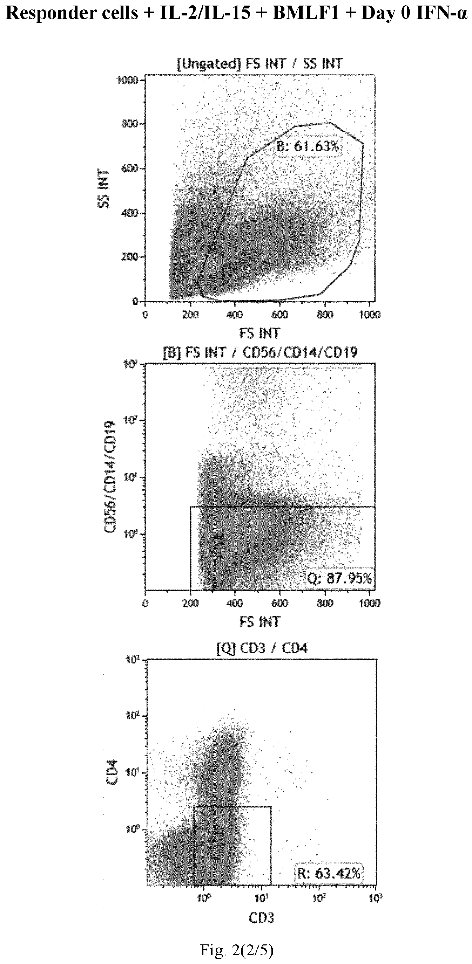

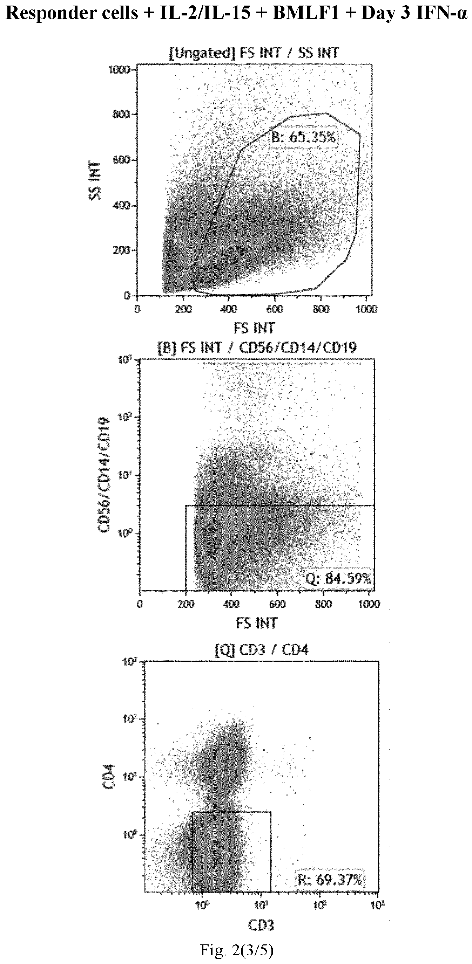

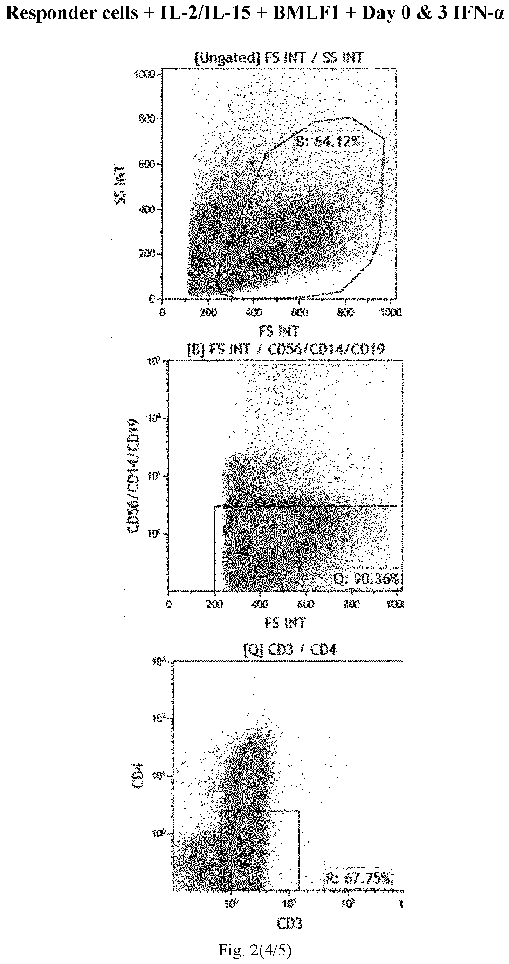

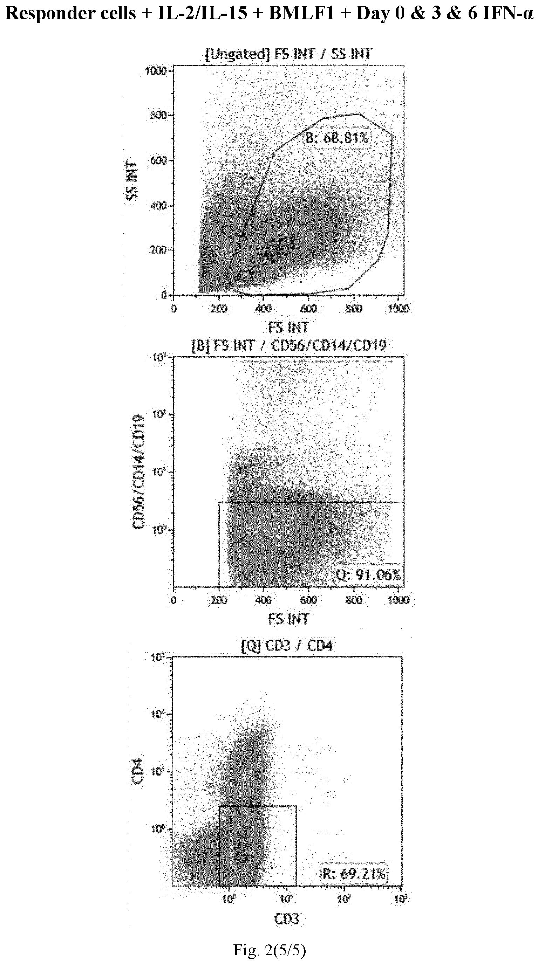

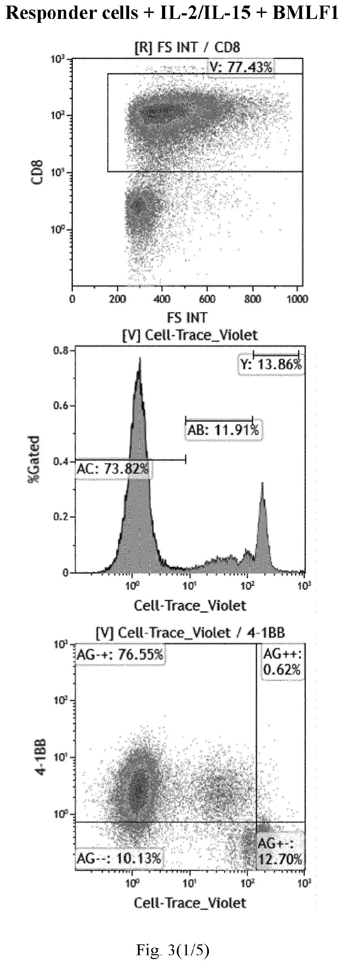

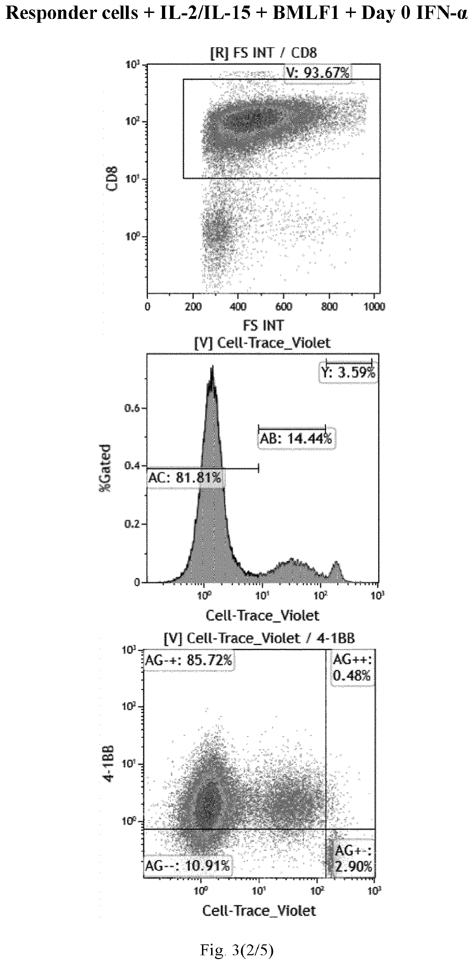

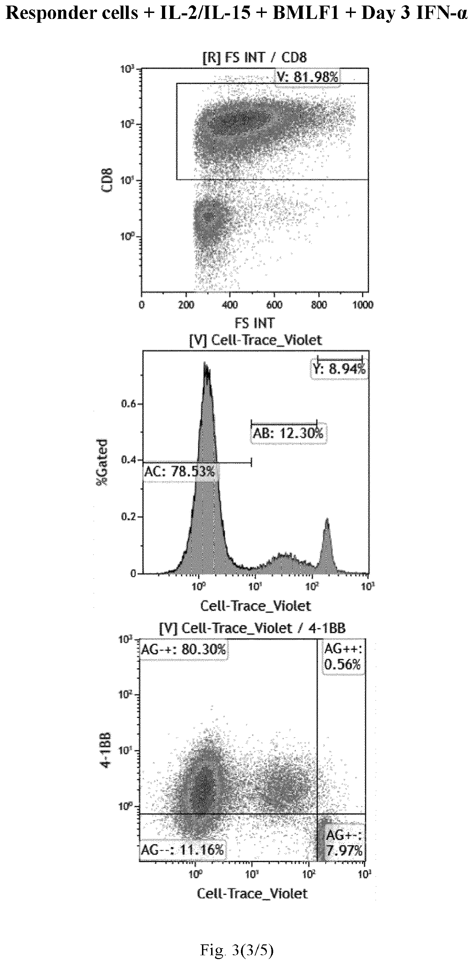

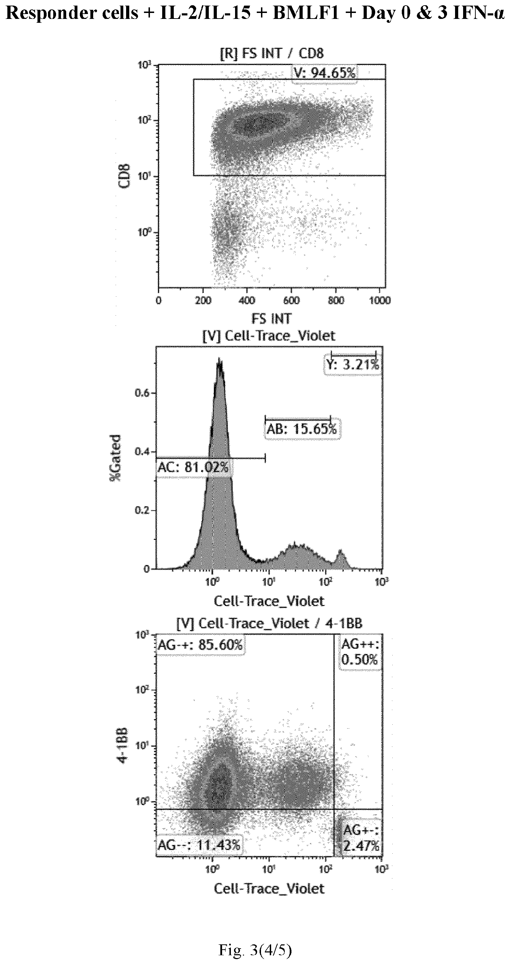

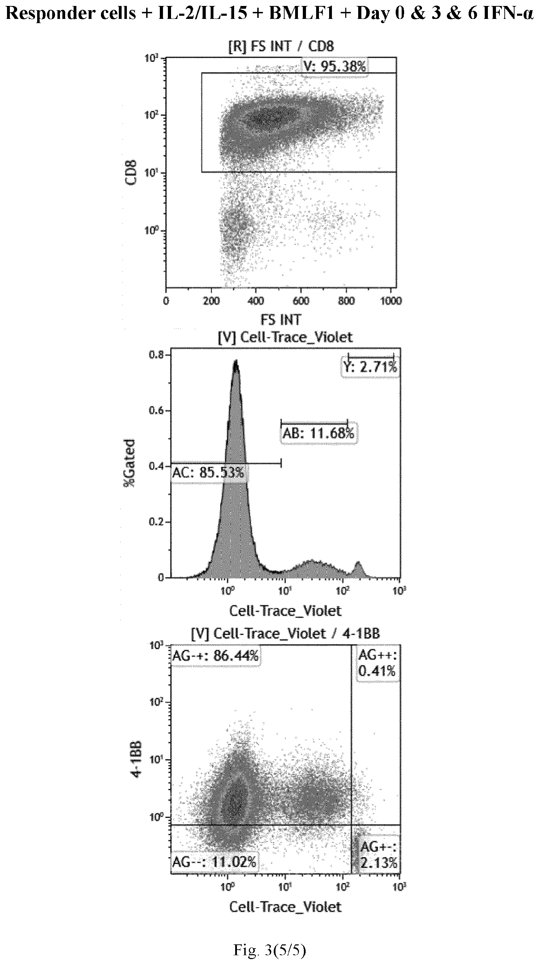

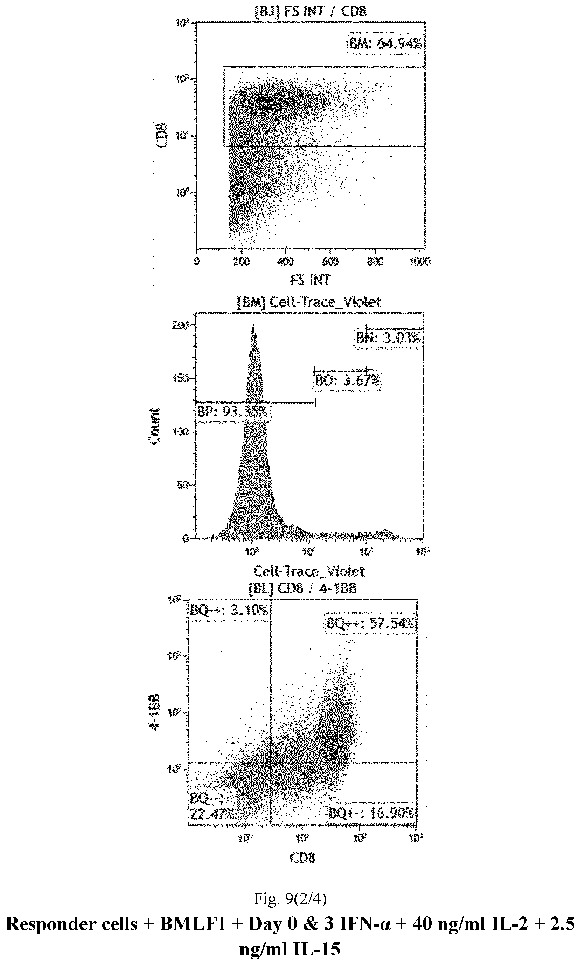

Please see Figs. 2 to 4. Figs. 2 to 4 illustrate addition of IFN-α on day 0 and day 3 promoted 4-1BB⁺CD8 T cells expansion. Responder PBMCs were labeled with Cell-Trace Violet, then cultured in AIM-V medium and HPL (4%), hIL-2 (40 ng/ml) and hIL-15 (2.5 ng/ml) for 9 days. In each particular group, responder PBMCs were treated with 5 ng/ml IFN-α on day0, day 3, day 0 plus day3 or day 0 plus day3 and day 6. On day 9, total cells were harvested and stained with mAbs against 4-1BB-PE, CD8-APC-Alexa Flour 700, CD56-APC- Cy7, CD14-APC-Alexa Flour 750, CD19APC-Alexa Flour 750, and CD3-Krome Orange. Samples acquisition via Navios Flow Cytometer and data analysis via Kaluza software. Data statistical analysis was performed by Dunnett's multiple comparison test. *, P<0.05.

As shown in Figs. 2 to 4, this example is an experiment for different addition timing for IFN-α to confirm the effect of IFN-α action time on the proportion of CD8 T cells and 4-1BB⁺ CD8 T cells. IFN-α inducement on day 0/3, i.e. IFN-α added on both day 0 and day 3, can induce more CD8 T cells and 4-1BB⁺ CD8 T cells in percentage comparing to addition of IFN-α only on day 0 or day 3. However, there is no difference comparing to addition of IFN-α on day 0/3/6. Further calculation of the increasing percentage difference between each group and the group without IFN-α, it can also be seen that the IFN-α inducement time must be at least on day 0/3. Therefore, the IFN-α addition timing was set as day 0/3 for the following experiments.

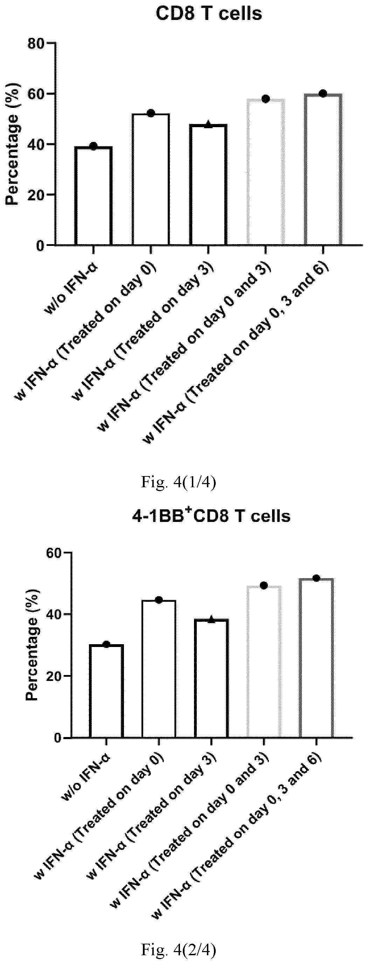

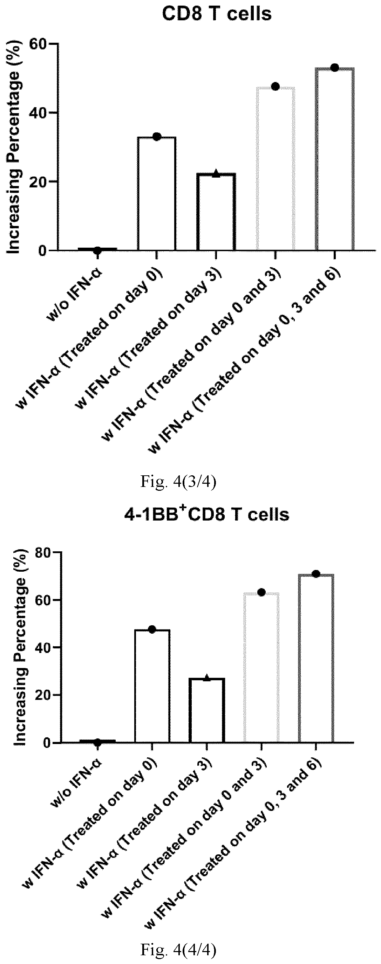

Further, as shown in Fig. 4, the treatment with IFN-α obviously induce the differentiation of CD8 T cells. However, the increasing percentage of the treatment applied on day 3 is significantly lower than the groups including treatment on day 0, which means that the treatment with IFN-α performs better in an early stage of the T cell differentiation.

### Example 3

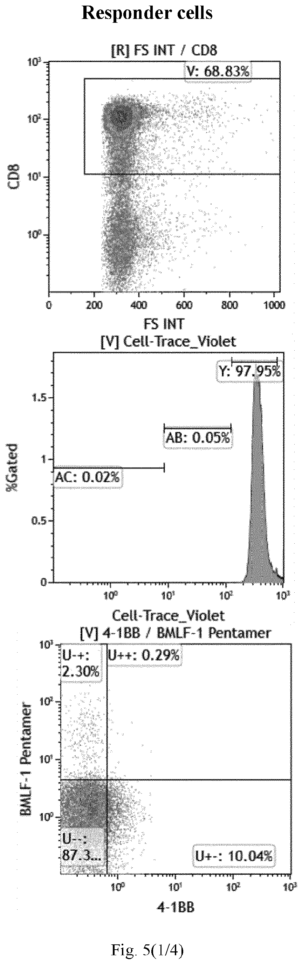

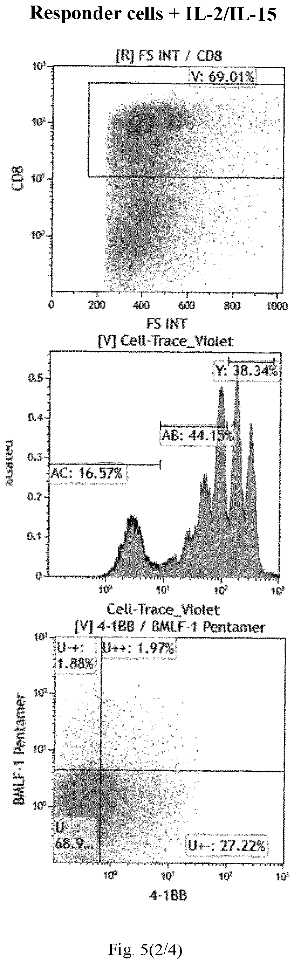

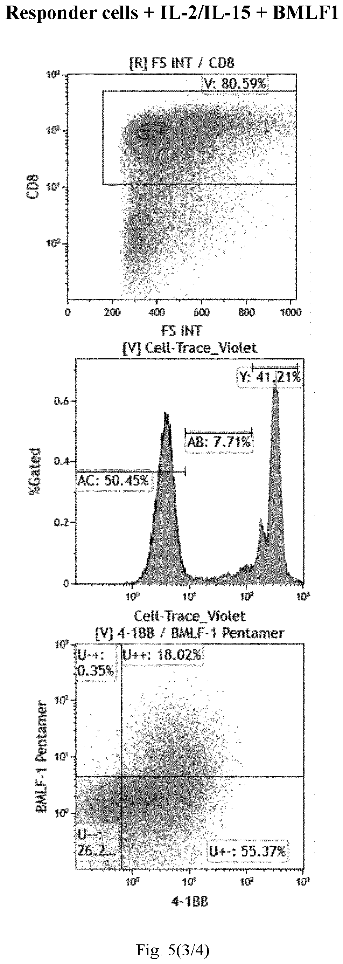

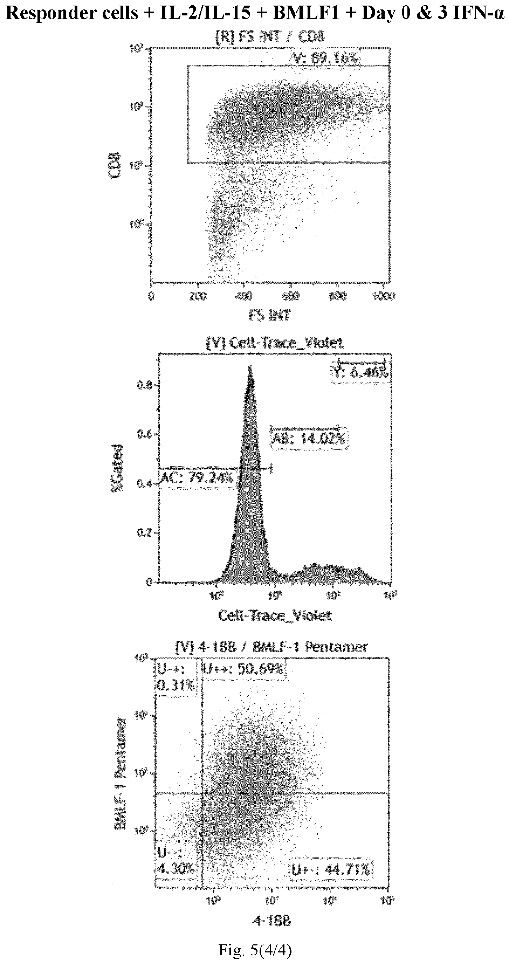

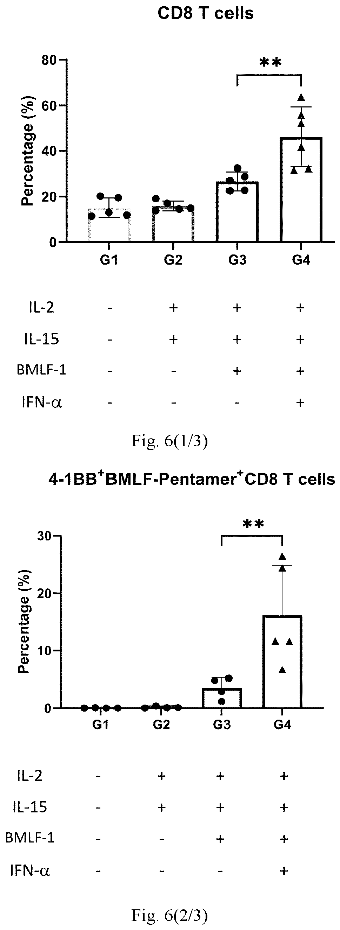

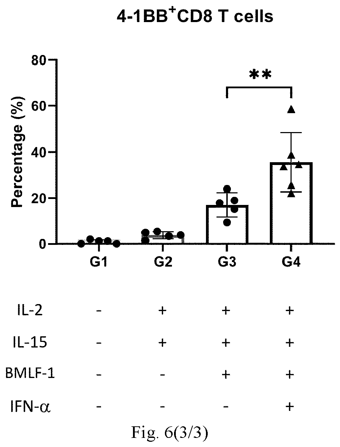

Please see Figs. 5 and 6, Figs. 5 and 6 illustrate Addition of IFN-α on day 0 and day 3 promoted 4-1BB⁺BMLF1-specific CD8 T cells expansion. Responder PBMCs were labeled with Cell-Trace Violet, then cultured in AIM-V medium and HPL (4%), hIL-2 (40 ng/ml) and hIL-15 (2.5ng/ml) for 9 days. In the particular group, responder PBMCs were treated with 5 ng/ml IFN-α day 0 and 3. On day 9, total cells were harvested and stained with mAbs against 4-1BB-PE, BMLF1-pentamer-APC, CD8-APC-Alexa Flour 700, CD56-APC-Cy7, CD14-APC-Alexa Flour 750, CD19APC-Alexa Flour 750, and CD3-Krome Orange. Samples acquisition via Navios Flow Cytometer and data analysis via Kaluza software. Data statistical analysis was performed by Dunnett's multiple comparison test. *, P<0.05.

As shown in Figs. 5 and 6, the combination of IL-2 and IL-15 and BMLF1 induces more differentiation of PBMC to CD8 T cells, and the combination of IL-2 and IL-15 and BMLF1 and IFN-α on day 0/3 is significantly better than that.

### Example 4

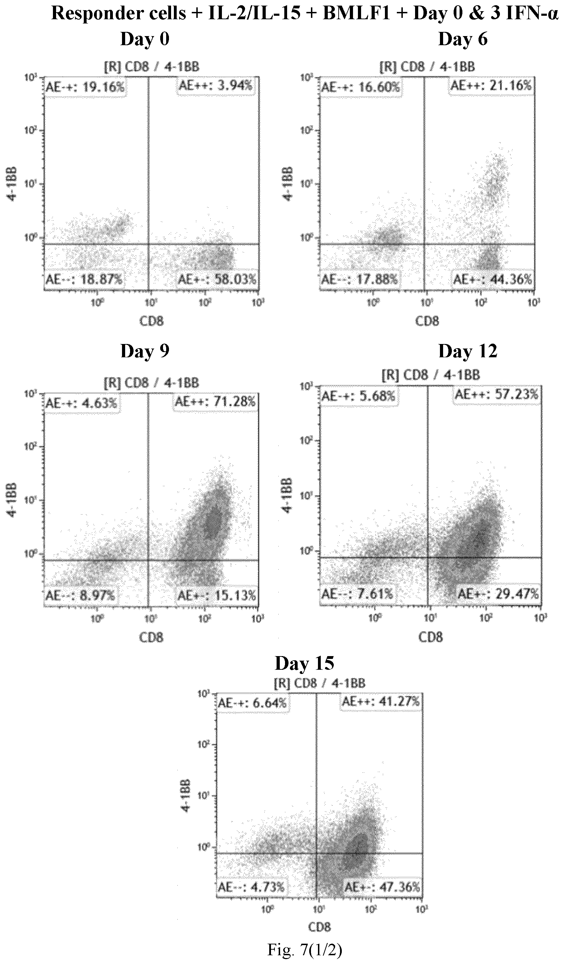

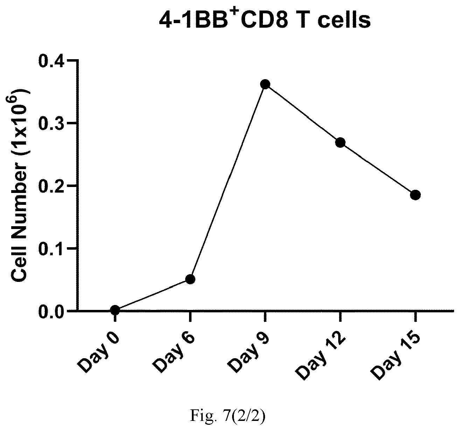

Please see Fig. 7. Fig. 7 illustrates nine-day culture period revealed the best 4-1BB⁺BMLF1-specific CD8 T cell expansion. Responder PBMCs were cultured in AIM-V medium and HPL (4%), BMI,F 1 peptide (10 µg/ml), hIL-2 (40 ng/ml), hIL-15 (2.5 ng/ml) and IFN-α (5 ng/ml). On day 9 and day 12, total cells were harvested and stained with mAbs against 4-1BB-PE, BMLF1-pentamer-APC, CD8-APC-Alexa Flour 700, CD56-APC-Cy7, CD14-APC-Alexa Flour 750, CD19APC-Alexa Flour 750, and CD3-Krome Orange. Samples acquisition via Navios Flow Cytometer and data analysis via Kaluza software.

Example 4 is a time-lapse experiment. It is shown that the addition of IFN-α on day 0/3 may induce most 4-1BB⁺ T cell on day 9. Therefore, the culture time thereof is set on day 9 for the following experiments.

### Example 5

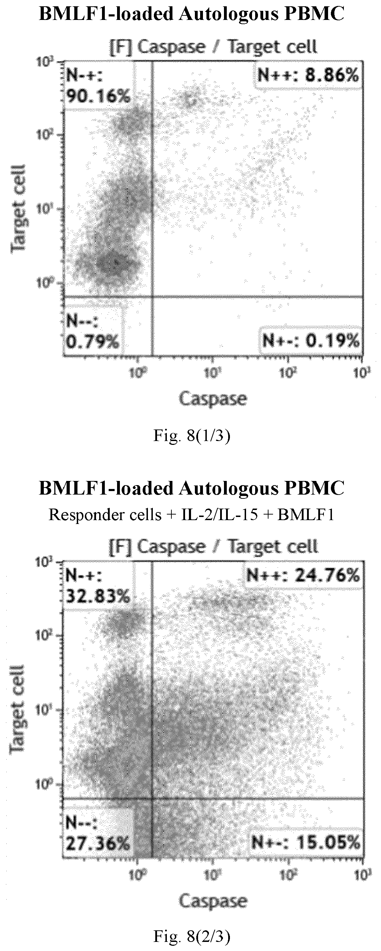

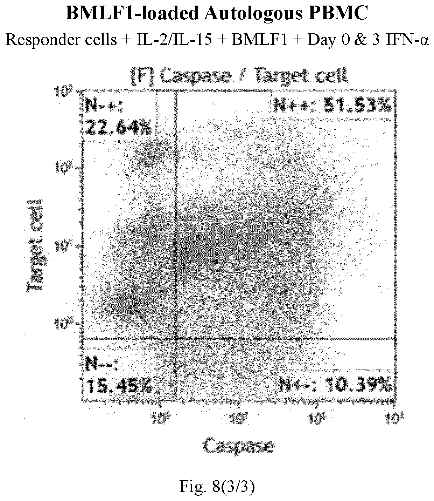

Please see Fig. 8, Fig. 8 illustrates addition of IFN-α on day 0 and day 3 displayed the best T-dependent cytotoxicity of 4-1BB⁺BMLF1-specific CD8 T cells. Evaluation of cytotoxicity of EBaT8 was performed by PanToxilux kit (OncoImmunin, Inc.). Autologous BMLF1-loaded PBMCs served as a target cell and stained with TFL4 under the optimal concentration for 50 minutes. Co-incubation of TFL-4 labeled target cell and responder cells with the caspase substrate under 37°C for 30 minutes. Harvested the cells and analyzed the signal of TFL-4+substrate+ via flow cytometry.

As shown in Fig. 8, the group having addition of IFN-α on day 0 and day 3 display higher caspase, which means the cytotoxicity of the differentiated cell induced by day 0/3 is higher than the other two groups.

### Example 6

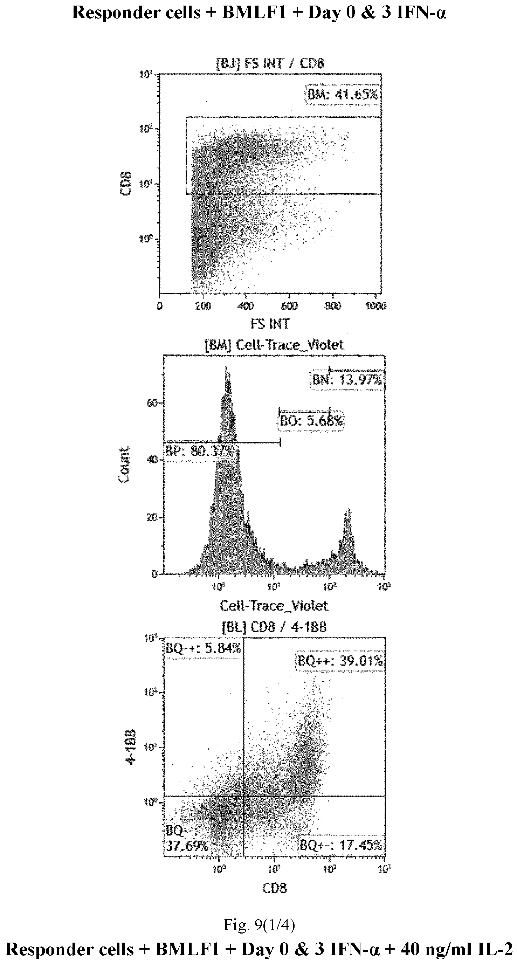

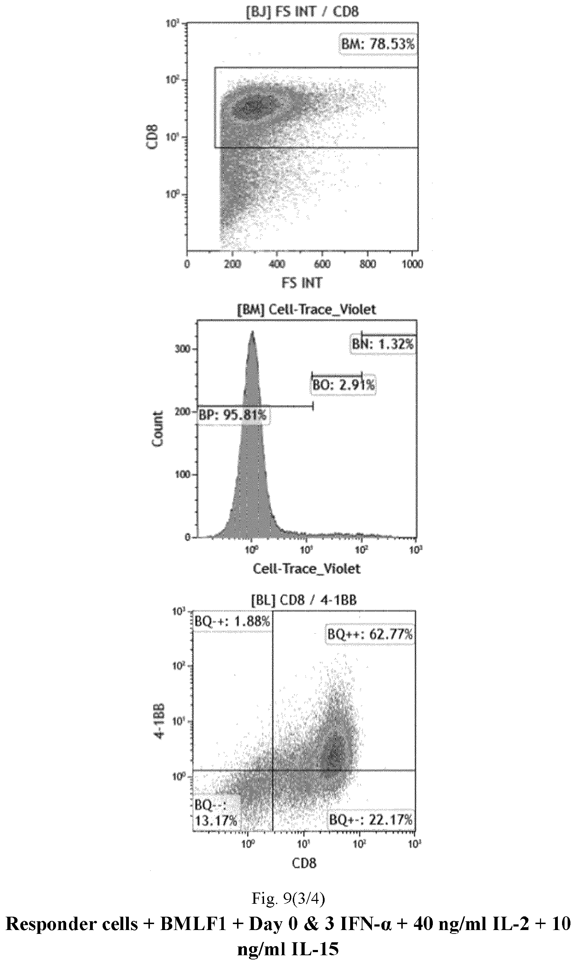

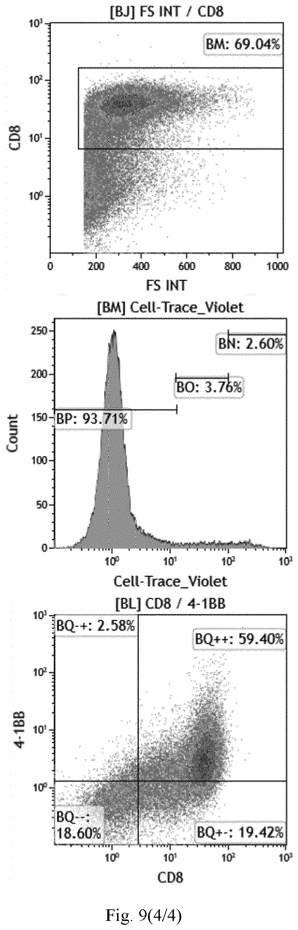

Please see Fig. 9, Fig. 9 illustrates different IL-2 synergized with IL-15 to promote BMLF1-induced EBV specific CD8 T cell responses. Responder PBMCs were labeled with Cell-Trace Violet, then cultured in AIM-V medium and HPL (4%), hIL-2 (40 ng/ml) and IFN-α (5ng/ml) for 9 days. In each particular group, responder PBMCs were treated with 2.5 ng/ml or 10 ng/ml hIL-15 for nine days. On day 9, total cells were harvested and stained with mAbs against 4-1BB-PE, BMLF1-pentamer-APC, CD8-APC-Alexa Flour 700, CD56-APC-Cy7, CD14-APC-Alexa Flour 750, CD19APC-Alexa Flour 750, and CD3-Krome Orange. Samples acquisition via Navios Flow Cytometer and data analysis via Kaluza software. Data statistical analysis was performed by Dunnett's multiple comparison test. *, P<0.05.

As shown in Fig. 9, IL-2 is added in 3 groups with 40 ng/ml and IL-15 is added with 2.5 ng/ml and 10 ng/ml in 2 groups, respectively. The result showed that IL-2 + IL-15 + BMLF-1 and day 0/3 IFN-α is the best culture condition.

### Working Example 1

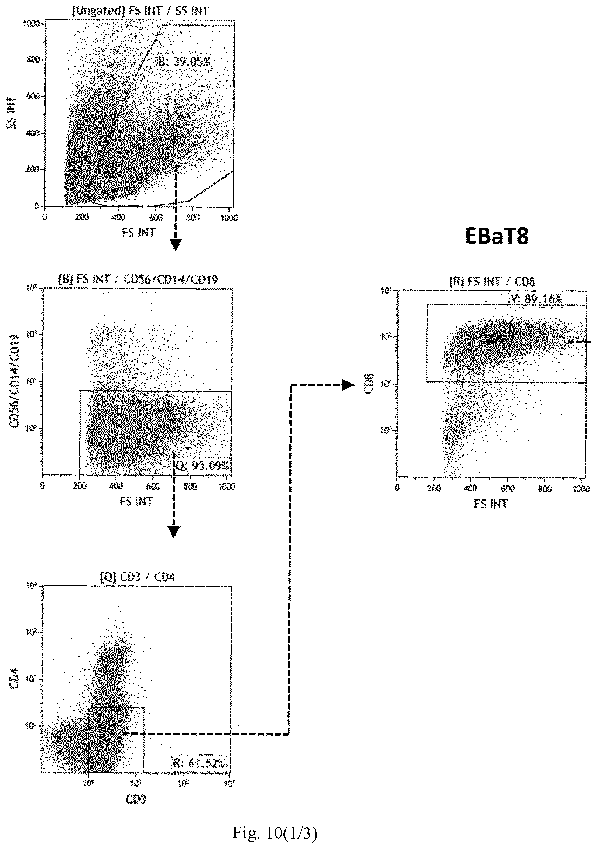

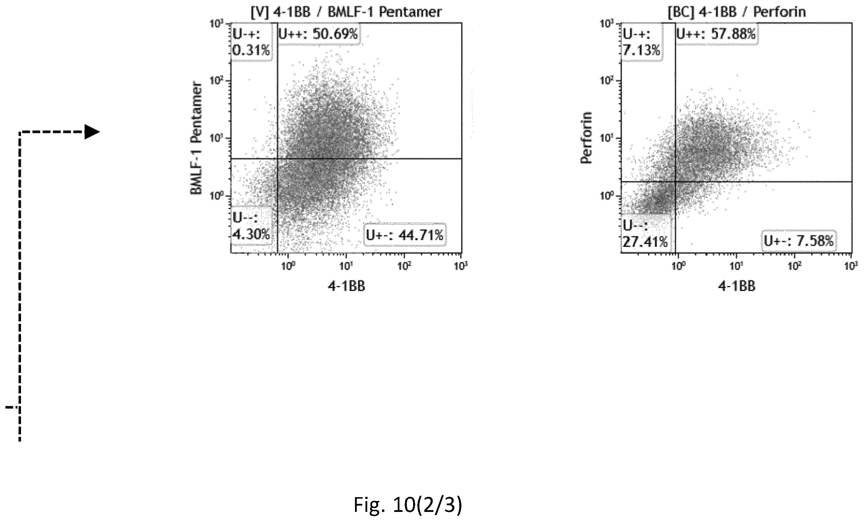

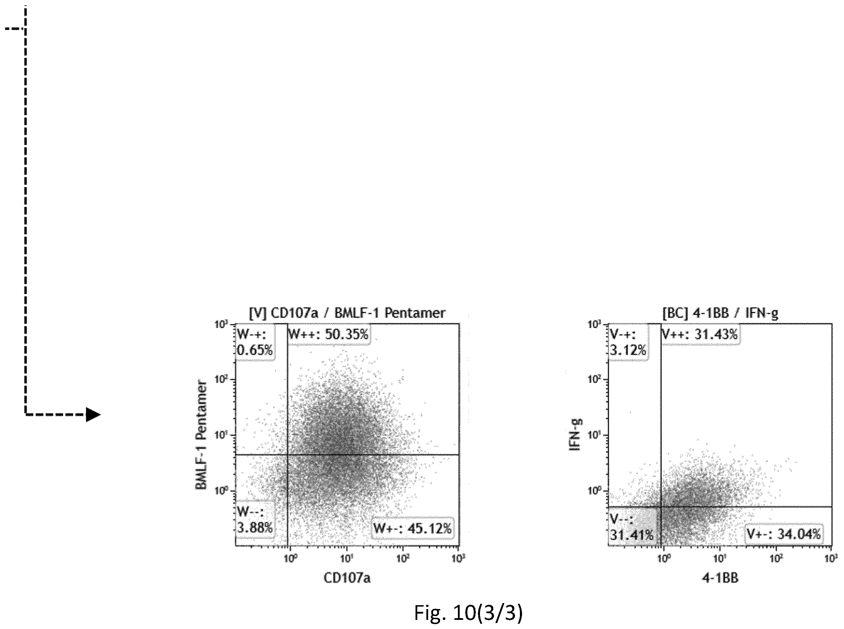

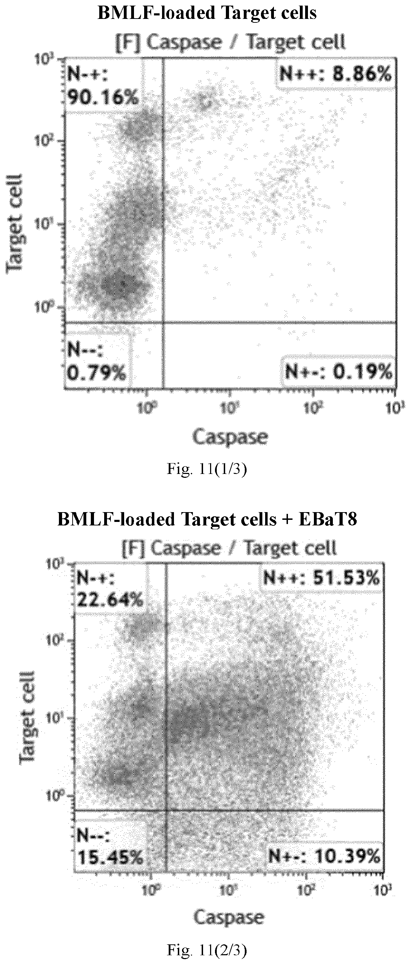

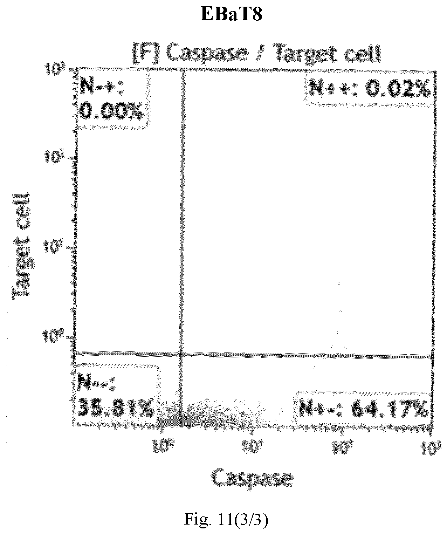

Fig. 10 shows the result of phenotypic analysis and the release of degranulation molecules by flow cytometry. Please see Fig. 11, Fig. 11 illustrates functionality analysis of EBaT8. Evaluation of cytotoxicity of EBaT8 was performed by PanToxilux kit (OncoImmunin, Inc.). Autologous BMLF1-loaded PBMCs served as a target cell and stained with TFL4 under the optimal concentration for 50 minutes. Co-incubation of TFL-4 labeled target cell and EBaT8 or bystander cytokine activated T cells with the caspase substrate under 37°C for 30 minutes. Harvested the cells and analyzed the signal of TFL-4+substrate+ via flow cytometry.

As shown in Fig. 11, the cultured cell (EbaT8) with IL-2 + IL-15 + BMLF-1 and addition of IFN-α on day 0/3 has the probability of killing BMLF-loaded target cells.

### Working Example 2

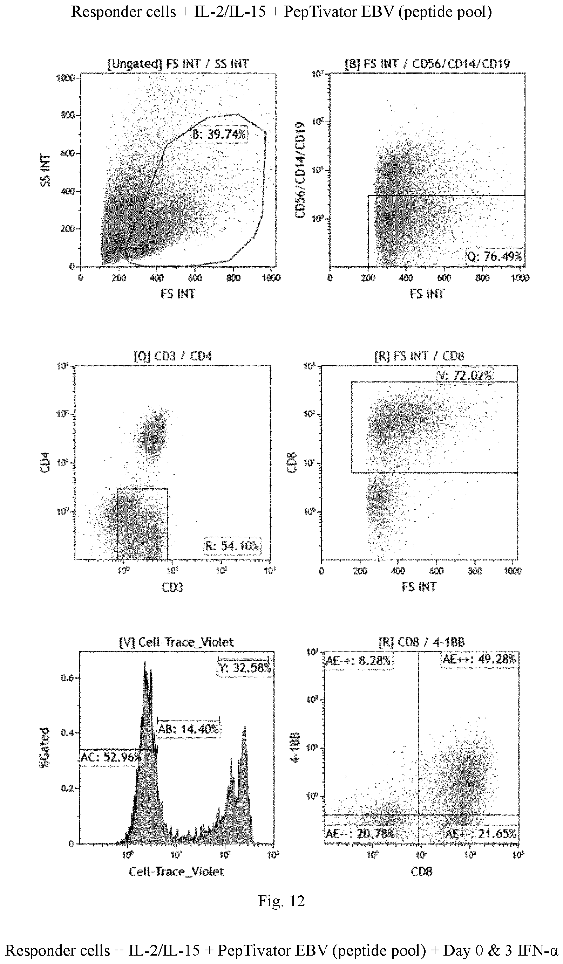

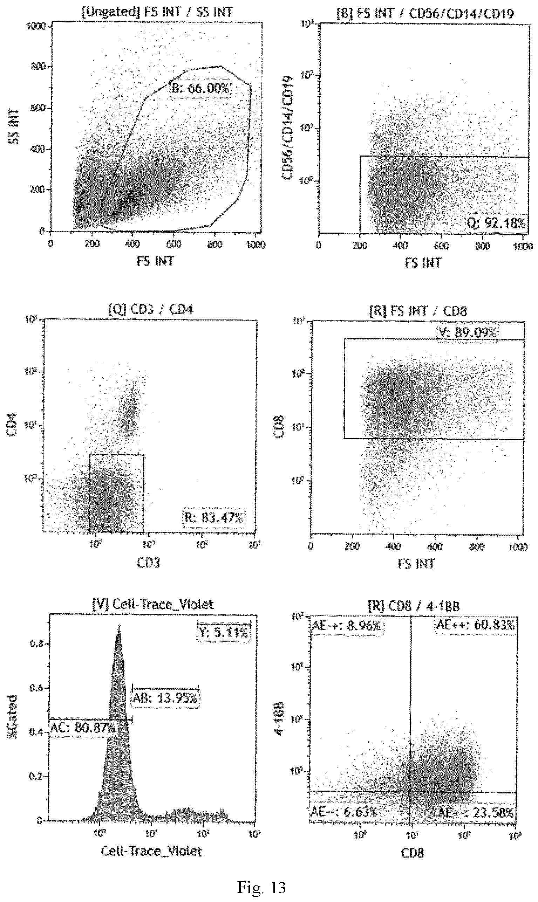

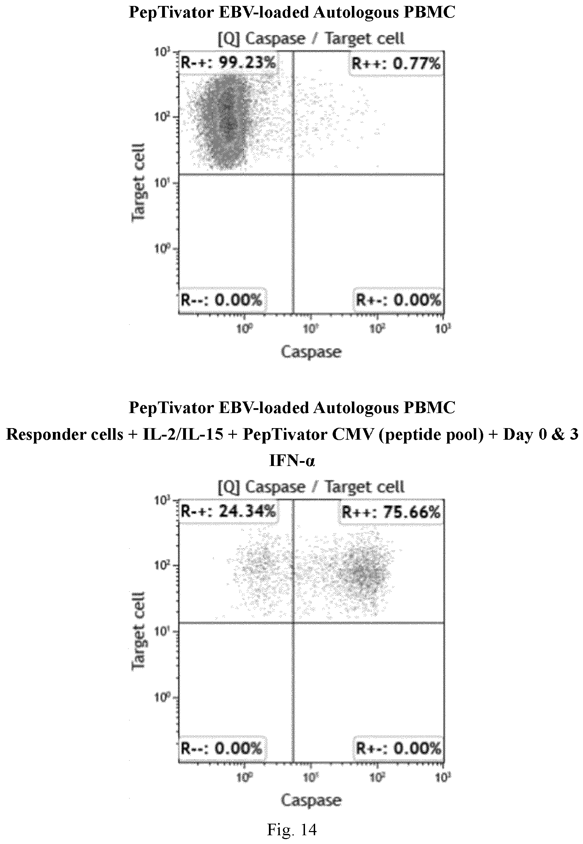

Please see the results in Figs. 12 to 13. Responder PBMCs were labeled with Cell-Trace Violet, then cultured in AIM-V medium and HPL (4%), PepTivator EBV (0.5 mg/ml), hIL-2 (40 ng/ml), hIL-15 (2.5 ng/ml) and IFN-α (5 ng/ml). On day 9, total cells were harvested and stained with mAbs against 4-1BB-PE, CD8-APC-Alexa Flour 700, CD56-APC-Cy7, CD14-APC-Alexa Flour 750, CD19APC-Alexa Flour 750, and CD3-Krome Orange. Samples acquisition is performed via Navios Flow Cytometer and data are analyzed via Kaluza software. Further see the result in Fig. 14, evaluation of the cytotoxicity of EBaT8 was performed by PanToxilux kit (OncoImmunin, Inc.). Autologous PepTivator EBV-loaded PBMCs were served as a target cell and stained with TFL4 under the optimal concentration for 50 minutes. Co-incubation of TFL-4 labeled target cell and EBaT8 with the caspase substrate under 37°C for 30 minutes. The cells are harvested and analyzed by the signal of TFL-4+substrate+ via flow cytometry.

As shown in Figs. 12 to 14, the PepTivator EBV peptide pool is capable of inducing EBV-specific T cell (EBaT8) expansion in the presence of IFN-α, and EBaT8 generated from PepTivator EBV peptide pool and IFN-α exhibited T-dependent cytotoxicity. The result indicates that the 4-1BB⁺ CD8 T cell may be induced by EBV peptide pool which comprises different sequence and can generate T cell-dependent killing. Thus, the feasibility of clinical trials thereof may be improved.

### Working Example 3

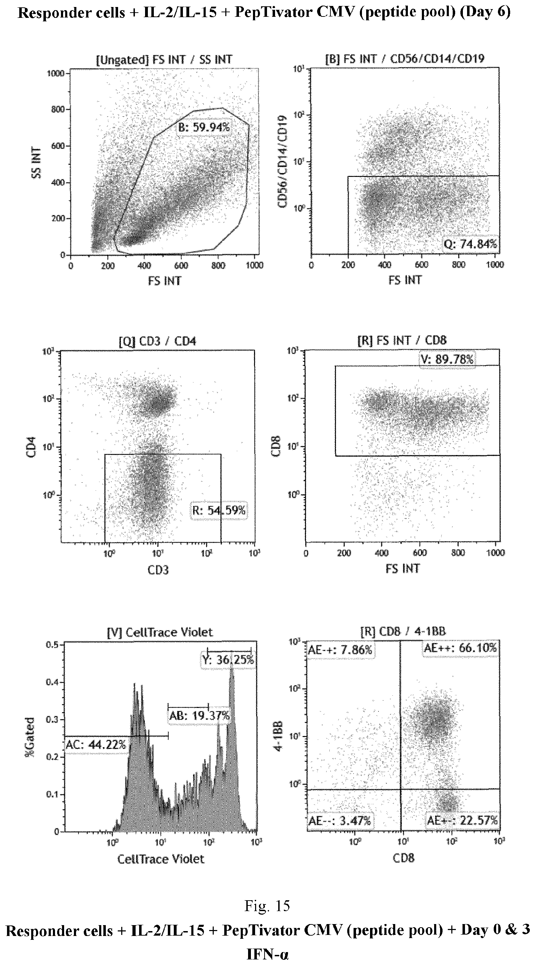

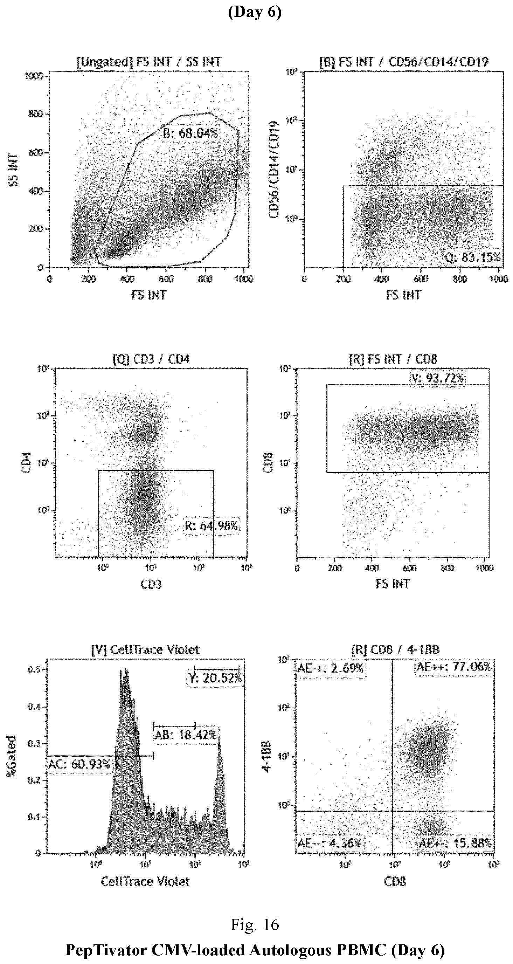

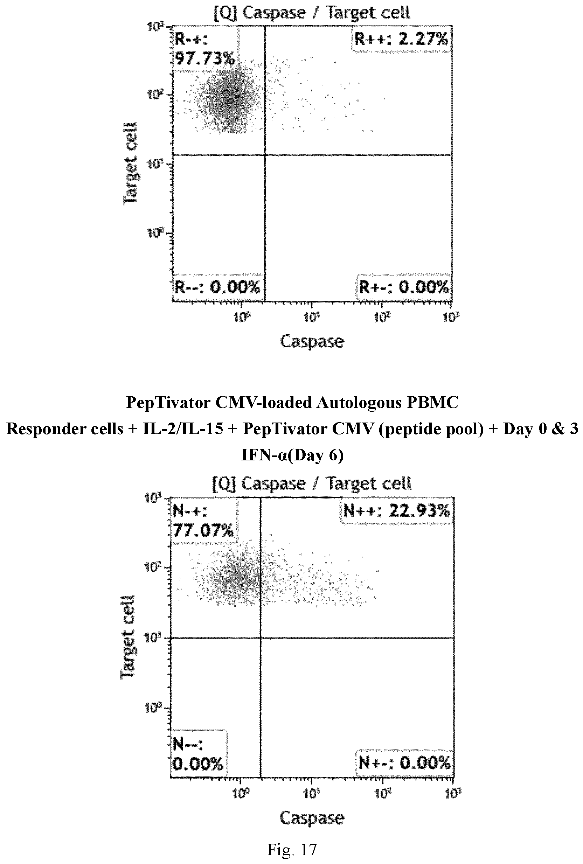

Please see the result in Figs. 15 and 16. Responder PBMCs were labeled with Cell-Trace Violet, then cultured in AIM-V medium and HPL (4%), PepTivator CMV (0.5 mg/ml), hIL-2 (40 ng/ml), hIL-15 (2.5 ng/ml) and IFN-α (5 ng/ml). On day 6, total cells were harvested and stained with mAbs against 4-1BB-PE, CD8-APC-Alexa Flour 700, CD56-APC-Cy7, CD14-APC-Alexa Flour 750, CD19APC-Alexa Flour 750, and CD3-Krome Orange. Samples acquisition via Navios Flow Cytometer and data analysis via Kaluza software. Further see the result in Fig. 17, evaluation of cytotoxicity of EBaT8 was performed by PanToxilux kit (OncoImmunin, Inc.). Autologous PepTivator EBV-loaded PBMCs served as target cells and stained with TFL4 under the optimal concentration for 50 minutes. Co-incubation of TFL-4 labeled target cell and EBaT8 with the caspase substrate under 37°C for 30 minutes. Harvested the cells and analyzed the signal of TFL-4+substrate+ via flow cytometry.

As shown in Figs. 15 to 17, on day 6, PepTivator CMV peptide pool is capable of induce CMV-specific T cell expansion in the presence of IFN-α, and CMV-specific T cells generated from PepTivator CMV peptide pool and IFN-α exhibited T-dependent cytotoxicity. The result indicates that the 4-1BB⁺ CD8 T cell may also be induced by peptide pool-CMV with IL-2 + IL-15 and the addition of IFN-α on day 0/3 and can generate T cell-dependent killing, which means that CMV, HPV or other tumor-associated antigen may also be used to induce the antigen-specific T cell amplification with the method described herein.

The above description is only exemplary but not limiting. Any equivalent modification or change made without departing from the spirit and scope of the present invention shall be included in the scope defined by the claims.

## Claims

1. A method for of inducing an antigen-specific T cell, comprising:
culturing a peripheral blood mononuclear cell (PBMC) in a first culture medium comprising an antigenic peptide, IL-2, IL-15 and IFN-α to obtain a first cell population;
culturing the first cell population in a second culture medium comprising IL-2, IL-15 and IFN-α to obtain a second cell population; and
culturing the second cell population in a third culture medium comprising IL-2 and IL-15 to obtain the antigen-specific T cell.

2. The method of claim 1, wherein the antigenic peptide is from a cancer or a virus in a concentration of 1 µg/mL to 1 mg/mL.

3. The method of claim 2, wherein the cancer comprises nasopharyngeal cancer, bladder cancer, biliary cancer, bone cancer, brain tumor, breast cancer, cervical cancer, colorectal cancer, colon cancer, esophageal cancer, epidermal carcinoma, gastric cancer, gastrointestinal stromal tumor (GIST), glioma, hematopoietic tumors of lymphoid lineage, hepatic cancer, non-Hodgkin's lymphoma, Kaposi's sarcoma, leukemia, lung cancer, lymphoma, intestinal cancer, melanoma, myeloid leukemia, pancreatic cancer, prostate cancer, retinoblastoma, ovary cancer, renal cell carcinoma, spleen cancer, squamous cell carcinoma, thyroid cancer, or thyroid follicular cancer.

4. The method of claim 2, wherein the cancer is Epstein-Barr virus (EBV)-associated cancer.

5. The method of claim 4, wherein the cancer is EBV-positive gastric cancer, EBV-positive cervical cancer, EBV-positive Burkitt's lymphoma, EBV-positive T cell lymphoma, EBV-positive breast cancer, EBV-positive leiomyosarcoma, EBV-positive smooth muscle tumor, EBV-positive Hodgkin lymphoma, EBV-positive nasopharyngeal cancer, or EBV-positive post-transplant lymphoproliferative disorder (PTLD).

6. The method of claim 4, wherein the antigenic peptide comprising BMI,F-1, EBV peptide pool or CMV peptide pool.

7. The method of any one of claims 1-6, wherein the first culture medium, the second culture medium and the third culture medium is cultured for 1-3 days, respectively.

8. The method of any one of claims 1-6, wherein the IL-2 is in a concentration from 1 to 500 ng/mL.

9. The method of any one of claims 1-6, wherein the IL-15 is in a concentration from 1 to 100 ng/mL.

10. The method of any one of claims 1-6, wherein the IFN-α is in a concentration from 1 to 100 ng/mL.

11. The method of any one of claims 1-6, wherein the first culture medium, the second culture medium and the third culture medium further comprises AIM-V medium, respectively.

12. The method of any one of claims 1-6, wherein the first culture medium, the second culture medium and the third culture medium further comprises platelet lysate, preferably human platelet lysate (HPL), in a concentration of 1 to 10 wt% based on the culture medium.

13. Use of a composition comprising a therapeutically effective amount of the antigen-specific T cell manufactured by the method of any one of claims 1-12 and a pharmaceutically acceptable carrier for preventing or treating cancer or virus infection in a subject in need thereof.

14. Use of a composition comprising a therapeutically effective amount of the antigen-specific T cell manufactured by the method of any one of claims 1-12 and a pharmaceutically acceptable carrier for manufacture of a medicament for preventing or treating cancer or virus infection in a subject in need thereof.

15. A composition for use in preventing or treating cancer or virus infection in a subj ect in need thereof, comprising a therapeutically effective amount of the antigen-specific T cell manufactured by the method any one of claims 1-12 and a pharmaceutically acceptable carrier.
